# EUROPÄISCHE PATENTSCHRIFT

(11) **EP 2 323 717 B1**
(45) Veröffentlichungstag und Bekanntmachung des Hinweises auf die Patenterteilung: **01.05.2013**
(21) Anmeldenummer: 09775758.7
(22) Anmeldetag: 31.07.2009
(51) Int. Cl.: A61M 5/20

(54) **AUTOMATISCHE INJEKTIONSVORRICHTUNG FÜR DIE VERABREICHUNG EINER FESTEN DOSIS**
AUTOMATIC INJECTION DEVICE FOR ADMINISTERING A FIXED DOSE
DISPOSITIF D'INJECTION AUTOMATIQUE POUR L'ADMINISTRATION D'UNE DOSE FIXE

(30) Priorität: 11.08.2008 DE 102008037310
(43) Veröffentlichungstag der Anmeldung: 25.05.2011
(73) Patentinhaber: Tecpharma Licensing AG, 3401 Burgdorf (CH)
(72) Erfinder: HIRSCHEL, Juerg, CH-3007 Bern (CH); MOSER, Ulrich, CH-3412 Heimiswil (CH); TSCHIRREN, Markus, CH-3422 Kirchberg (CH); VOGT, Patrick, CH-4704 Niederbipp (CH)
(86) Internationale Anmeldenummer: PCT/CH2009/000267
(87) Internationale Veröffentlichungsnummer: WO 2010/017650

(56) Entgegenhaltungen:
- WO-A-2005/044344
- WO-A-2005/097238
- DE-A1-102004 060 146
- US-A- 4 902 279
- US-A1- 2004 039 337
- US-A1- 2007 021 720

## Beschreibung

Die vorliegende Erfindung bezieht sich auf eine Vorrichtung zur Selbstverabreichung einer festen Dosis einer bestimmten Substanz. Eine solche Vorrichtung wird auch als fixed-dose Pen bezeichnet und ermöglicht eine einfache einmalige Selbstverabreichung einer in einer Ampulle oder Spritze innerhalb der Injektionsvorrichtung gelagerten Substanz.

Eine Injektionsvorrichtung zur Abgabe einer fest eingestellten Dosis, welche zum Beispiel einmalig voreingestellt werden kann, ist aus der DE 10 2006 038 103 A1 der Anmelderin bekannt.

Es ist eine Aufgabe der vorliegenden Erfindung eine Injektionsvorrichtung für eine einmalige Abgabe einer fest vorgegebenen Menge einer Substanz vorzuschlagen, welche einfach aufgebaut und bedient werden kann.

Diese Aufgabe wird durch die Injektionsvorrichtung gemäß Anspruch 1 gelöst. Vorteilhafte Ausführungsformen sind in den abhängigen Ansprüchen definiert.

Eine Injektionsvorrichtung weist ein Gehäuse auf, an welchem die Injektionsvorrichtung durch den Benutzer gehalten werden kann, wobei eine Kolbenstange vorzugsweise innerhalb des Gehäuses vorgesehen ist, welche auf einen Stopfen einer Ampulle oder Spritze drücken kann, welche vorzugsweise ebenfalls innerhalb der Injektionsvorrichtung angeordnet ist, um durch den Druck auf den Stopfen eine innerhalb der Ampulle oder Spritze enthaltene Substanz aus dieser zu verdrängen und vorzugsweise durch eine Nadel abzugeben. Die Kolbenstange weist mindestens ein bewegbares oder auslenkbares Halteelement auf, wie zum Beispiel einen Federarm mit radial ausragendem Eingriffbereich, welches die Kolbenstange relativ zu dem Gehäuse und bevorzugt an dem Gehäuse möglichst unverschiebbar in einer festen Position hält. Des weiteren ist an oder in der Injektionsvorrichtung ein verschiebbares Sicherungselement, zum Beispiel eine Hülse, vorzugsweise eine Abdeckhülse, vorgesehen, welches relativ zum Gehäuse der Injektionsvorrichtung verschiebbar ist und welches das mindestens eine Halteelement der Kolbenstange in einer festen Position relativ zum Gehäuse hält, wenn das Sicherungselement oder die Hülse in einer ersten Position ist. Das mindestens eine Halteelement der Kolbenstange kann freigegeben werden, wenn das Sicherungselement oder die Hülse aus der ersten Position verschoben ist, so dass die Kopplung zwischen der Kolbenstange und der Injektionsvorrichtung oder dem Gehäuse der Injektionsvorrichtung gelöst und vorzugsweise die Kolbenstange innerhalb der Injektionsvorrichtung bewegt oder verschoben werden kann, um zum Beispiel die Injektion auszulösen oder durchzuführen.

Vorzugsweise ist das Sicherungselement oder die Hülse eine Abdeckhülse, welche sich im distalen Bereich der Injektionsvorrichtung befindet und aus der Injektionsvorrichtung herausragt. Diese Abdeckhülse ist vorteilhaft relativ zur Injektionsvorrichtung oder dem Gehäuse der Injektionsvorrichtung verschiebbar, also zum Beispiel in das Gehäuse der Injektionsvorrichtung in axiale Richtung einschiebbar, wenn die Injektionsvorrichtung auf eine Oberfläche, wie zum Beispiel Haut, aufgesetzt wird und ist vorteilhaft verdrehgesichert im Gehäuse der Injektionsvorrichtung gelagert. Wird das Gehäuse der Injektionsvorrichtung in Richtung auf die Haut geschoben, kann die Abdeckhülse in das Gehäuse oder in die Injektionsvorrichtung, vorzugsweise nach Überwindung einer von zum Beispiel einer lösbaren Rastverbindung verursachten Mindestgegenkraft eingeschoben werden, das heißt das Gehäuse der Injektionsvorrichtung bewegt sich auf die Oberfläche zu. Vorzugsweise ist an dem Gehäuse mindestens eine Rippe oder Rast- oder Haltestufe vorgesehen, an welcher das mindestens eine Halteelement gehalten werden kann. Die Rippe oder Haltestufe kann zum Beispiel eine Fläche haben, auf welcher die Längsachse oder Axialachse der Injektionsvorrichtung senkrecht steht und ist bevorzugt abgeschrägt, so dass das mindestens eine Halteelement der Kolbenstange leichter freigegeben werden kann, wenn es nicht mehr von der verschiebbaren Abdeckhülse gehalten oder fixiert wird. Das mindestens eine Halteelement der Kolbenstange hat vorteilhaft einen Haltebereich, der korrespondierend zur Anlagefläche der Haltestufe ausgebildet, also zum Beispiel ebenfalls leicht abgeschrägt ist.

Vorzugsweise ist ein Injektionsfederelement vorgesehen, welches sich zum Beispiel an dem Gehäuse oder einem mit dem Gehäuse bevorzugt fest verbundenen oder verrasteten Element, wie zum Beispiel einer Endkappe, abstützt und welches auf die Kolbenstange in distale Richtung drückt. Das Injektionsfederelement ist vorzugsweise in Ausgangsstellung der Injektionsvorrichtung gespannt und weist eine Kraft auf, die ausreicht, um das mindestens eine Halteelement aus der Halte- oder Eingriffsposition zu bringen, wenn das Halteelement nicht mehr durch die Sicherung oder Abdeckhülse gehalten oder arretiert wird, so dass dann durch die Kraft des auf die Kolbenstange drückenden Injektionsfederelements die Kolbenstange relativ zur Injektionsvorrichtung oder relativ zum Gehäuse bewegbar wird und durch die Kraft des Injektionsfederelements in Richtung auf den Stopfen der Ampulle oder Spritze geschoben wird, um den Ausschütt-Vorgang zu initiieren.

Vorteilhaft ist die Abdeckhülse in dem Gehäuse der Injektionsvorrichtung oder innerhalb der Injektionsvorrichtung verdrehgesichert, zum Beispiel durch eine axial verlaufende Nut und/oder einen Steg der Abdeckhülse, welche mit einem korrespondierenden Steg und/oder einer axialen Nut der Injektionsvorrichtung oder des Gehäuses zusammenwirkt oder eine Axialführung bildet.

Vorzugsweise ist eine Abdeckelementfeder in der Injektionsvorrichtung vorgesehen, welche sich vorteilhaft gegen die Injektionsvorrichtung oder deren Gehäuse abstützt und welche auf die verschiebbare Abdeckhülse drückt, so dass die Abdeckhülse durch die Abdeckhülsenfeder in distale Richtung mit einem Druck beaufschlagt werden kann. Wird die Injektionsvorrichtung auf eine Oberfläche aufgesetzt und auf diese zu bewegt, so kann die Abdeckhülse gegen die die Kraft der Abdeckhülsenfeder in die Injektionsvorrichtung eingeschoben werden, was zu einer Kompression und somit Spannung der Abdeckhülsenfeder führt.

In der Injektionsvorrichtung ist vorteilhaft eine Ampulle oder Spritze vorgesehen, welche vorzugsweise fest mit dem Gehäuse verbunden ist oder fest in dem Gehäuse der Injektionsvorrichtung zum Beispiel durch einen oder mehrere Anschläge und/oder durch die auf die Ampulle oder den Stopfen der Ampulle drückende Kolbenstange gehalten wird. Die Spritze weist vorzugsweise eine Einstechnadel am distalen Ende auf, welche zum Beispiel durch eine abnehmbare Nadelschutzkappe gesichert sein kann. Die Nadel ist vorzugsweise so in der Injektionsvorrichtung angeordnet, dass diese bei ausgeschobener Abdeckhülse von dieser umgeben wird und bei zurückgeschobener Abdeckhülse freiliegt, so dass bei Aufdrücken der Injektionsvorrichtung auf eine Oberfläche die Abdeckhülse eingeschoben werden kann und die dann freigelegte Injektionsnadel einstechen kann. Vorteilhaft erfolgt das Einstechen manuell durch Aufdrücken der Injektionsvorrichtung auf eine Oberfläche, so dass zum Beispiel für den Einstechvorgang kein Knopf oder Auslöseelement betätigt werden muss. Dabei ist zum Beispiel die Abdeckhülse so in der Injektionsvorrichtung gelagert, dass bei dem Aufdrücken der Injektionsvorrichtung auf eine Oberfläche, auf welcher die Abdeckhülse dann auf- oder anliegt, eine Anfangskraft überwunden werden muss, um ein Einschieben der Abdeckhülse zu bewirken, wodurch sichergestellt werden kann, dass der Einstechvorgang rasch erfolgt, nachdem die Anfangskraft überwunden und die Abdeckhülse durch den nachfolgenden Druck rasch zurückgeschoben und somit die Nadel rasch eingestochen wurde.

Gemäß einem weiteren Aspekt bezieht sich die Erfindung auf eine Injektionsvorrichtung, welche aus wenigen Einzelteilen zusammengesetzt werden kann, wie zum Beispiel nur drei oder vier Einzelteilen, welche zum Beispiel aus Kunststoff geformt werden können, wie zum Beispiel Gehäuse, Abdeckhülse, Kolbenstange und optional Endabdeckung oder Endkappe. Dabei ist zum Beispiel nur eine einzige Feder für die Injektion und optional eine weitere Feder für die Abdeckhülse vorgesehen.

Erfindungsgemäß kann eine Injektionsvorrichtung geschaffen werden, mit welcher eine Einzeldosierung, also zum Beispiel eine einzige Ausschüttung, möglich ist, wobei die Injektionsvorrichtung bevorzugt nach erfolgter Ausschüttung automatisch blockiert. Das Ausschüttvolumen ist dabei bevorzugt durch den Inhalt der in der Injektionsvorrichtung enthaltenen Ampulle oder Spritze fest vorgegeben. Die Ausschüttung erfolgt vorzugsweise automatisch nach dem Einstechen, das heißt es ist vorteilhaft kein weiterer Betätigungsmechanismus mehr zu betätigen, damit der Ausschüttvorgang nach dem Einstechen beginnt. Dieser Ausschüttvorgang läuft vollautomatisch nach dem Einstechvorgang ab. Vorzugsweise verfügt die Injektionsvorrichtung über einen Sicherheits- Nadelschutz und verriegelt oder schützt die Injektionsnadel nach dem Gebrauch, das heißt nach erfolgter Injektion, indem beispielsweise die Abdeckhülse automatisch in axialer Richtung über die Injektionsnadel wieder ausgeschoben wird.

Gemäß einem weiteren Aspekt bezieht sich die Erfindung auf ein Verfahren zum Zusammensetzten oder Montieren einer Injektionsvorrichtung aus zwei und vorzugsweise genau zwei Unterbaugruppen, wobei eine einzusetzende Ampulle oder Spritze vorzugsweise nicht als Unterbaugruppe angesehen werden soll. Bei dem Zusammensetzen der Injektionsvorrichtung aus den zwei Unterbaugruppen wird eine Injektionsfeder erst bei dem Zusammensetzen der Unterbaugruppen gespannt. Die Injektionsfeder ist vorzugsweise Bestandteil einer der Unterbaugruppen und liegt im nicht zusammengesetzten Zustand in entspannter, also beispielsweise nicht in komprimierter oder gespannter Form, vor. Hierdurch kann sichergestellt werden, dass Verschlechterungs- oder Alterungsprozesse bedingt durch den von einer gespannten Injektionsfeder ausgehenden Druck nicht schon beginnend ab dem Zusammenbau der Unterbaugruppen auftreten.

Vorzugsweise weist eine der beiden Baugruppen das Gehäuse der Injektionsvorrichtung und optional auch eine zum Beispiel in das Gehäuse eingesetzte relativ dazu verschiebbare Abdeckhülse auf, welche beispielsweise aus dem distalen Bereich des Gehäuses herausragen kann. Die zweite Unterbaugruppe weist vorzugsweise eine Kolbenstange, eine Injektionsfeder und ein Injektionsfederabstützelement auf, wobei die Injektionsfeder zwischen dem Injektionsfederabstützelement und der Kolbenstange gespannt und zum Beispiel komprimiert werden kann. Dabei kann beispielsweise die Kolbenstange oder das Kolbenstangenelement relativ zum Injektionsfederabstützelement verschoben und zum Beispiel geführt durch das Injektionsfederabstützelement in dieses eingeschoben werden, so dass die Injektionsfeder zwischen der Kolbenstange und dem Injektionsfederabstützelement gespannt oder zusammengedrückt werden kann. Beim Zusammensetzen der Injektionsvorrichtung kann eine Spritze oder Ampulle zwischen die erste Unterbaugruppe und die zweite Unterbaugruppe vor dem Zusammensetzen eingebracht werden. Beispielweise kann die Spritze oder Ampulle in die erste Unterbaugruppe eingeschoben werden. Die zweite Unterbaugruppe kann anschließend in die erste Unterbaugruppe eingesetzt oder eingeschoben werden. Dabei wird vorteilhaft die Kolbenstange oder das distale Ende der Kolbenstange auf die Spritze oder Ampulle, zum Beispiel auf einen in der Spritze oder Ampulle verschiebbaren Stopfen, gedrückt. Da in dem Ausgangszustand aus der Ampulle oder Spritze zum Beispiel auf Grund eines aufgesetzten Nadelschutzelements kein Fluid oder keine Substanz entweichen kann, wird beim Einsetzen der zweiten Unterbaugruppe in die erste Unterbaugruppe die Kolbenstange relativ zum Injektionsfederabstützelement zum Beispiel durch Druck des Stopfens verschoben, so dass erst dadurch die Injektionsfeder gespannt wird. Optional kann auch eine Abdeckhülsenfeder zwischen der ersten und der zweiten Unterbaugruppe oder an der zweiten Unterbaugruppe vorgesehen sein, welche beispielsweise zwischen dem Injektionsfederabstützelement einerseits und der Abdeckhülse andererseits im zusammengesetzten Zustand liegt, so dass die Abdeckhülse beim Einschieben in das Gehäuse eine Spannung oder Kompression der Abdeckhülsenfeder bewirkt.

Die Erfindung wird nachfolgend anhand eines bevorzugten Ausführungsbeispiels beschrieben. Es zeigen
- Fig. 1: eine Injektionsvorrichtung im Ausgangszustand in Querschnittsansicht;
- Fig. 2: die in Figur 1 gezeigte Injektionsvorrichtung in Explosionsansicht vor dem Zusammensetzen,
- Fig. 3: die in Figur 2 gezeigte Injektionsvorrichtung im teilweise zusammengesetzten Zustand; und
- Fig. 4A bis 4C: den Ablauf einer Injektion mit der Injektionsvorrichtung.

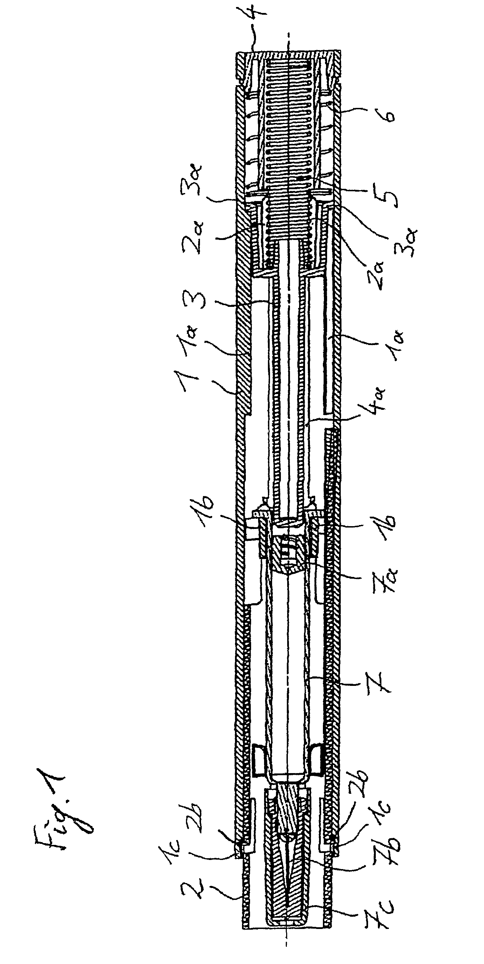

Figur 1 zeigt eine Injektionsvorrichtung mit einem Gehäuse 1, welches an seiner Innenseite in axiale Richtung verlaufende Rippen oder Stege 1a aufweist. An einer proximalen Seite der jeweiligen Rippe 1a liegt jeweils ein Halteelement 3a in Form eines Federarms mit radial nach außen weisendem Eingriff an, wobei die Haltelemente 3a an einer Kolbenstange 3 befestigt sind und diese in der gezeigten axialen Position halten. Im hinteren oder proximalen Bereich der Injektionsvorrichtung ist eine Injektionsfeder 5 vorgesehen, welche sich gegen eine in das Gehäuse 1 eingerastete Endkappe 4 abstützt und welche auf die Kolbenstange 3 und vorzugsweise die Halteelemente 3a der Kolbenstange 3 in distale Richtung drückt. Die Haltelemente 3a der Kolbenstange 3 werden durch Arme 2a der Abdeckhülse 2 bevorzugt formschlüssig oder mit kleinem Spiel in der Eingriffs- oder Halteposition an den Kanten der Stege 1a gehalten, so dass die Injektionsfeder 5 die Kolbenstange 3 nicht in distale Richtung schieben kann.

Auf distaler Seite der Kolbenstange 3 ist eine Ampulle oder Spritze 7 mit einem darin verschiebbaren Stopfen 7a vorgesehen, auf welchen die Kolbenstange 3 drücken kann. An der distalen oder Vorderseite der Spritze 7 ist eine Injektionsnadel 7b vorgesehen, auf welcher eine Nadelschutzkappe 7c aufgebracht ist, welche vor dem Gebrauch der Injektionsvorrichtung abgenommen wird. Die Abdeckhülse 2 weist vorzugsweise im Bereich der vorderen oder distalen Hälfte radial nach außenstehende Nasen 2b auf, welche in eine korrespondiere Nut oder Ausnehmung 1c des Gehäuses eingreifen, um einen Anfangswiderstand zu erzeugen, wenn die Abdeckhülse 2 durch einen Benutzer, welcher das Gehäuse 1 hält, gegen eine Oberfläche gedrückt wird. Wird die Kraft, mit welcher der Benutzer auf das Gehäuse 1 in distale Richtung drückt, größer, so lösen sich diese Halteelemente 1c aus ihrer Halteposition und geben die in der Injektionsvorrichtung 1 axial verschiebbar gelagerte Abdeckhülse schlagartig oder ruckartig frei, so dass diese sich in die Injektionsvorrichtung 1 einschieben kann, wodurch die Injektionsvorrichtung 1 ruckartig auf die Oberfläche gebracht und die Nadel 7b eingestochen wird.

Im hinteren oder proximalen Teil der Injektionsvorrichtung ist eine Abdeckhülsenfeder 6 vorgesehen, welche sich gegen das Gehäuse 1 der Injektionsvorrichtung oder im gezeigten Ausführungsbeispiel gegen die mit dem Gehäuse 1 verbundene oder verrastete Endkappe 4 abstützt und welche in distale Richtung auf die Abdeckhülse 2 drückt.

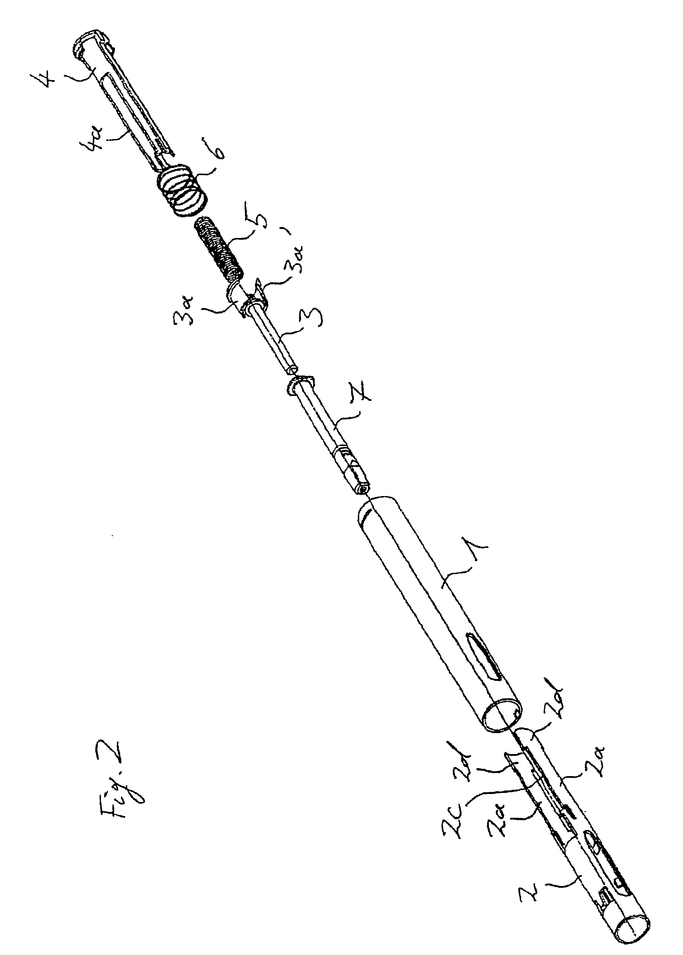

Figur 2 zeigt die in Figur 1 gezeigte Injektionsvorrichtung in Explosionsansicht, wobei die oben beschriebenen sieben Einzelkomponenten der Injektionsvorrichtung erkennbar sind. Die Abdeckhülse 2 weist in axiale Richtung weisend zwei Arme 2a mit proximalen End- oder Haltebereichen 2d und zwischen den Armen 2a axial zu den Haltebereichen 2d versetzt eine Freigabeaussparung 2c auf. Die Kolbenstange 3 hat zwei Halteelemente 3a und 3a', welche von den Haltebereichen 2d zum Beispiel auseinandergedrückt und/oder gegen ein Zusammendrücken gesichert werden können.

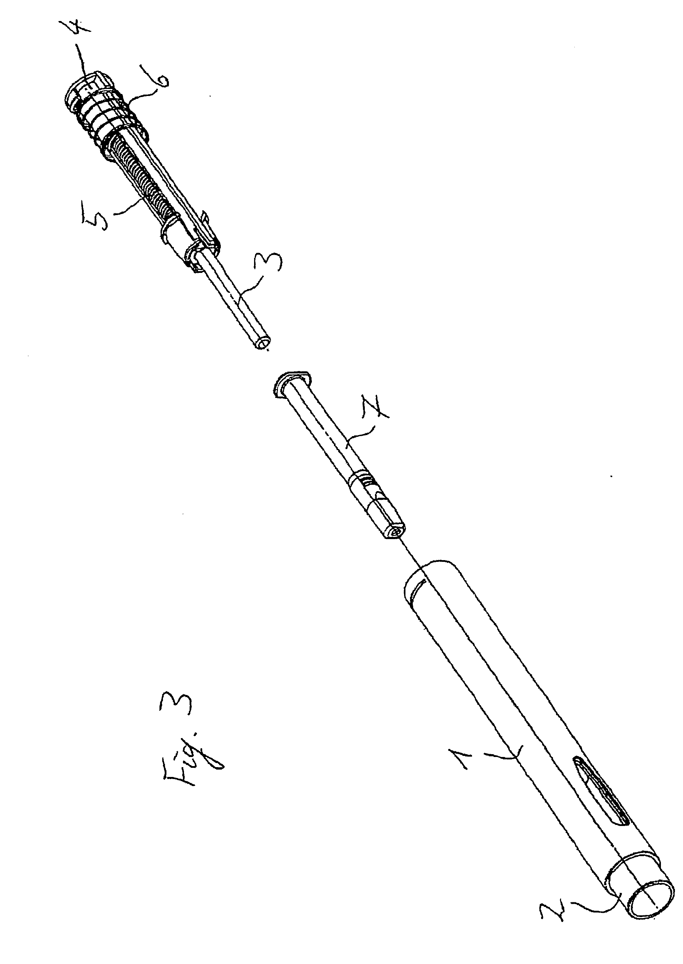

Figur 3 zeigt die teilweise zusammengesetzten Einzelkomponenten, wobei die Abdeckhülse 2 in das Gehäuse 1 eingesetzt wurde und die Kolbenstange 3, die Injektionsfeder 5 und die Endkappe 4 zu einer Baugruppe zusammengesetzt wurden.

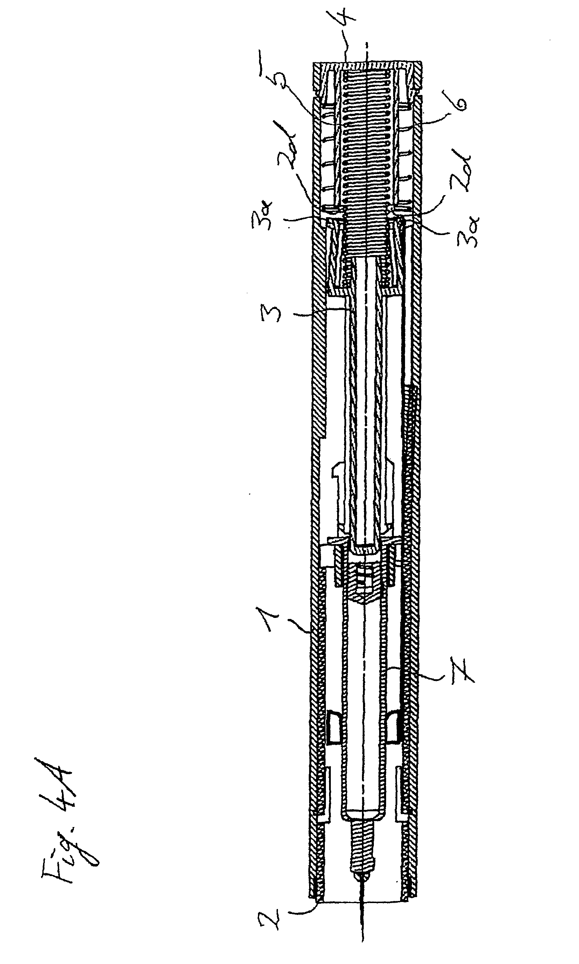

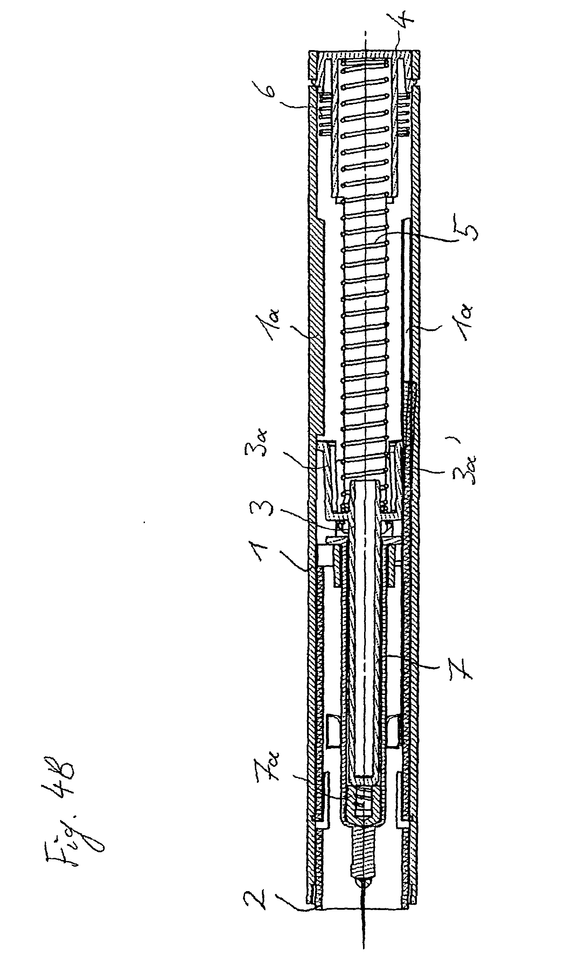

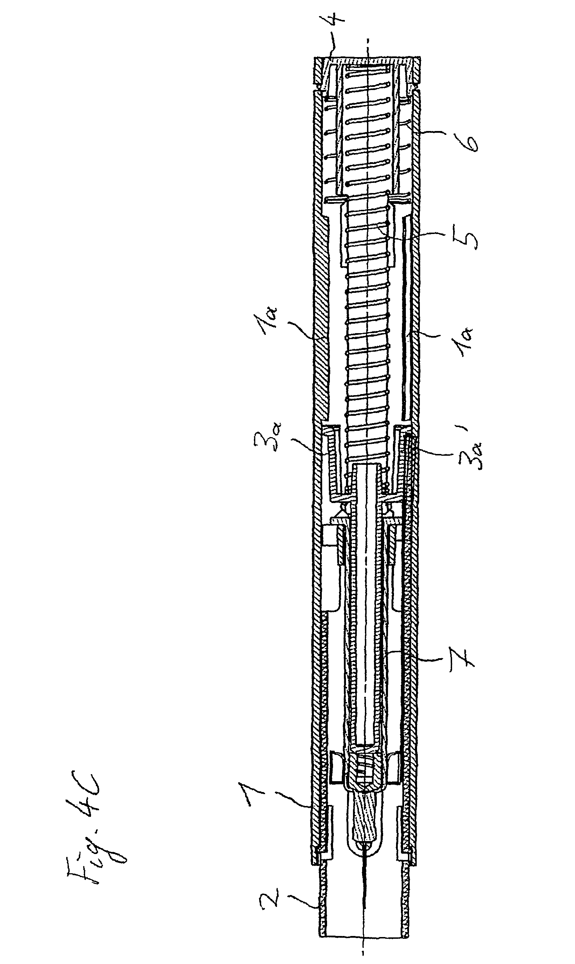

Die Figuren 1, 4A, 4B und 4C zeigen den Ablauf einer Injektion mit der Injektionsvorrichtung ausgehen von dem in Figur 1 gezeigten Auslieferungszustand.

Die Injektionsvorrichtung ist durch die Injektionsfeder 5, welche zwischen der Kolbenstange 3 und der Endkappe 4 gestaucht oder gespannt ist, geladen. Die Abdeckhülse 2 ist im Gehäuse 1 verdrehgesichert gelagert oder eingehängt und wird durch die Abdeckhülsenfeder 6 in die vordere oder distale Position gedrückt. Die Spritze 7 wird einerseits im Gehäuse 1 zum Beispiel an Anschlägen oder Halteelementen 1b gelagert, sowie zusätzlich durch Arme oder Rippen 2a in der Abdeckhülse 2 geführt und durch Stege 4a der Endkappe 4 gesichert. An der Kolbenstange 3 sind radial nach außen abstehende Federelemente 3a angebracht, welche an Rippen 1a im Gehäuse 1 anstehen und durch die proximale Seite 2d der Arme 2a der Abdeckhülse 2 gegen das Auslenken außerhalb der Halteposition radial nach innen gehindert werden.

Die Endkappe 4 ist in das Gehäuse 1 eingeschnappt oder eingerastet. Diese Schnapp- oder Rastverbindung ist so ausgelegt, dass sie die Kräfte der Injektionsfeder 5 und der Abdeckhülsenfeder 6 aufnehmen kann.

Figur 4A zeigt die Injektionsvorrichtung in einem ausgelösten Zustand nach Abnehmen der Abdeckhülse 7c von der Injektionsnadel 7b. Beim Einstechen wird die Abdeckhülse 2 so weit in die hintere Position geschoben bis die Federelemente 3a der Kolbenstange 3 durch eine Aussparung 2e in der Abdeckhülse 2 zum Auslenken freigestellt sind. Durch die Anschrägung an den Rippen 1a im Gehäuse 1 sowie an den Federelementen 3a der Kolbenstange 3 werden die Federelemente 3a der Kolbenstange 3 durch die Kraft der Injektionsfeder 5 ausgelenkt und somit die Ausschüttung automatisch ausgelöst. Die Injektionsfeder 5 bringt die Kraft auf, welche benötigt wird, um die Federelemente 3a auszulenken und somit die Kolbenstange 3 freizugeben, welche durch die Injektionsfeder 5 auf den Stopfen 7a der Spritze 7 gedrückt wird und diesen in die Spritze 7 einführt und somit die Ausschüttung automatisch auslöst.

Die Abdeckhülsenfeder 6 liegt an den proxialen Seiten 2d der Hülse 2 an und ist durch die eingeschobene Hülse 2 zusammengedrückt oder gestaucht worden.

Figur 4B zeigt die Injektionsvorrichtung nach dem Ausschüttvorgang. Durch das Entspannen der Injektionsfeder 5 wird die Kolbenstange 3 nach vorne in distale Richtung gestoßen. Die Kolbenstange 3 drückt auf den Stopfen 7a der Spritze 7 und schüttet die Spritze 7 komplett aus, bis der Stopfen 7a am Ende des Glaskörpers der Spritze 7 ansteht. Die beiden Federelemente 3a and der Kolbenstange 3 gleiten über die Rippen 1a des Gehäuses 1. Am Ende der Ausschüttung schnappt eines der beiden Federelemente 3a über das Ende einer oder mehrerer Rippen 1a im Gehäuse 1 und verursacht einerseits ein Geräusch nach dem Ausschütten, den sogenannten "Endklick", und sperrt andererseits die Kolbenstange 3 gegen ein Zurückschieben.

Das andere Federelement 3a' an der Kolbenstange 3 wird von einem als Sperrsteg wirkenden Arm 2a an der Abdeckhülse 2 im ausgelenkten Zustand gehalten.

Figur 4C zeigt die Injektionsvorrichtung im verriegelten Zustand nach der Abnahme vom Injektionsort.

Nach Beendigung der Injektion wird die Injektionsvorrichtung von der Injektionsstelle entfernt. Dabei wird die Abdeckhülse 2 von der Abdeckhülsenfeder 6 in die vordere oder distale Position gedrückt. Der Sperrsteg 2a der Abdeckhülse 2 verschiebt sich unter dem ausgelenkten Federelement 3a' an der Kolbenstange 3 soweit, dass das Federelement 3a' über das Ende Sperrstegs 2a schnappt und ein Geräusch, einen sogenannten "Klick", verursacht. In diesem Zustand ist die Injektionsvorrichtung blockiert. Die Abdeckhülse 2 kann nicht mehr eingedrückt werden, da diese von der Kolbenstange 3, welche an den zum Beispiel drei Rippen 1a im Gehäuse 1 ansteht, blockiert ist.

## Patentansprüche

1. Injektionsvorrichtung zur Verabreichung einer Substanz mit einem Gehäuse (1),
einer in dem Gehäuse (1) bewegbaren Kolbenstange (3),
an welcher mindestens ein Halteelement (3a) vorgesehen ist, das die Kolbenstange (3) relativ zu dem Gehäuse (1) hält
und mit einer verschiebbaren Sicherung, vorzugsweise einer Abdeckhülse (2),
welche innerhalb des Gehäuses (1) verschiebbar ist
und in einer ersten Position das mindestens eine Haltelement (3a) vorzugsweise formschlüssig in einer Halteposition relativ zum Gehäuse (1) hält
und in einer zweiten Position das mindestens eine Halteelement (3a) freigibt, **dadurch gekennzeichnet, dass**
die Abdeckhülse (2) einen Haltebereich (2d) aufweist, welcher das mindestens eine Halteelement (3a) in Position hält,
und eine Aussparung oder Ausnehmung (2c) aufweist,
um nach Verschieben der Abdeckhülse (2) das mindestens eine Halteelement (3a) freizugeben.

2. Injektionsvorrichtung nach Anspruch 1, wobei das mindestens eine Halteelement (3a) ein elastisches Element oder Federelement ist, welches vorzugsweise in radiale Richtung nach aussen vorgespannt ist und welches vorteilhaft mindestens einen Haltebereich oder abgeschrägten Bereich aufweist.

3. Injektionsvorrichtung nach einem der vorhergehenden Ansprüche, wobei das Gehäuse (1) der Injektionsvorrichtung mindestens eine Anlagekante oder einen Steg (1 a) aufweist, an welchem das mindestens ein Halteelement (3a) sich in einer ersten Position gegen eine Verschiebung in distale Richtung abstützen kann und/oder in einer zweiten in distale Richtung versetzen Position gegen eine Verschiebung in proximale Richtung abstützen kann.

4. Injektionsvorrichtung nach einem der vorhergehenden Ansprüche, wobei die Abdeckhülse(2) in der Injektionsvorrichtung oder in dem Gehäuse (1) verdrehgesichert gelagert ist.

5. Injektionsvorrichtung nach einem der vorhergehenden Ansprüche mit einer Injektionsfeder (5), welche sich das gegen das Gehäuse (1) oder gegen ein mit dem Gehäuse verbundenes Element (4) abstützt und welche in distale Richtung auf die Kolbenstange (3) drückt.

6. Injektionsvorrichtung nach einem der vorhergehenden Ansprüche mit einer Sicherungs- oder Abdeckhülsenfeder (6), welche sich gegen das Gehäuse (1) der Injektionsvorrichtung oder ein damit verbundenes Element (4) abstützt und welche auf die Sicherung oder Abdeckhülse (2) in distale Richtung eine Federkraft erzeugt oder drückt.

7. Injektionsvorrichtung nach einem der vorhergehenden Ansprüche mit einer Ampulle oder Injektionsspritze (7) mit einem darin einschiebbaren Stopfen (7a), wobei die Injektionsspritze (7) in dem Gehäuse (1) gelagert und/oder in der Abdeckhülse (2) verschiebbar geführt oder gelagert ist.

8. Verfahren zum Zusammensetzen einer Injektionsvorrichtung aus zwei Unterbaugruppen, wobei eine Injektionsfeder (5) erst bei dem Zusammensetzen der Unterbaugruppen gespannt wird, wobei die erste Unterbaugruppe ein Gehäuse (1) und die zweite Unterbaugruppe eine Kolbenstange (3), eine Injektionsfeder (5) und ein Injektionsfederabstützelement (4) aufweist, wobei die Injektionsfeder (5) zwischen der Kolbenstange (3) und dem Injektionsfederabstützelement (4) gespannt oder zusammengedrückt werden kann.

## Claims

1. An injection device for administering a substance comprising a housing (1),
a piston rod (3) which is movable in the housing (1) and
on which there is provided at least one holding element (3a) which holds the piston rod (3) relative to the housing (1),
and comprising a displaceable securing means, preferably a cover sleeve (2),
which is displaceable within the housing (1)
and which in a first position holds the at least one holding element (3a) preferably in positively locking relationship in a holding position relative to the housing (1)
and in a second position releases the at least one holding element (3a), **characterised in that**
the cover sleeve (2) has a holding region (2d) which holds the at least one holding element (3a) in position,
and has a hollow or recess (2c)
in order to release the at least one holding element (3a) after displacement of the cover sleeve (2).

2. An injection device according to claim 1 wherein the at least one holding element (3a) is an elastic element or spring element which is preferably biased outwardly in the radial direction and which advantageously has at least one holding region or bevelled region.

3. An injection device according to one of the preceding claims wherein the housing (1) of the injection device has at least one contact edge or a limb (1a) against which the at least one holding element (3a) can be supported in a first position to prevent a displacement in the distal direction and/or can be supported in a second position displaced in the distal direction to prevent displacement in the proximal direction.

4. An injection device according to one of the preceding claims wherein the cover sleeve (2) is mounted in the injection device or in the housing (1) in such a way as to be prevented from rotating.

5. An injection device according to one of the preceding claims comprising an injection spring (5) which is supported against the housing (1) or against an element (4) connected to the housing and which presses against the piston rod (3) in the distal direction.

6. An injection device according to one of the preceding claims comprising a securing or cover sleeve spring (6) which is supported against the housing (1) of the injection device or an element (4) connected thereto and which presses or produces a spring force on the securing means or cover sleeve (2) in the distal direction.

7. An injection device according to one of the preceding claims comprising an ampoule or injection syringe (7) with a stopper (7a) which can be pushed thereinto, wherein the injection syringe (7) is mounted in the housing (1) and/or is mounted or guided displaceably in the cover sleeve (2).

8. A method of assembling an injection device from two sub-units wherein an injection spring (5) is stressed only upon assembly of the sub-units, wherein the first sub-unit has a housing (1) and the second sub-unit has a piston rod (3), an injection spring (5) and an injection spring support element (4), wherein the injection spring (5) can be stressed or compressed between the piston rod (3) and the injection spring support element (4).

## Revendications

1. Dispositif d'injection pour l'administration d'une substance avec un boîtier (1), une tige de pison (3) mobile dans le boîtier (1),
sur laquelle au moins un élément de retenue (3a) est prévu, qui tient la tige de piston (3) par rapport au boîtier (1)
et comportant une sécurité pouvant coulisser, de préférence une gaine de recouvrement (2) qui peut coulisser dans le boîtier (1)
et maintient dans une première position au moins un élément de maintien (3a) de préférence par complémentarité de forme dans une position de retenue par rapport au boîtier (1)
et libère dans une deuxième position le au moins un élément de retenue (3a), **caractérisé en ce que**
la gaine de recouvrement (2) comporte une zone de retenue (2d) qui maintient le au moins un élément de retenue (3a) en position,
et présente un évidement ou une entaille (2c), pour libérer le au moins un élément de retenue (3a) après coulissement de la gaine de recouvrement (2),

2. Dispositif d'injection selon la revendication 1, dans lequel au moins un élément de retenue (3a) est un élément élastique ou un élément à ressort qui est précontraint de préférence en direction radiale vers l'extérieur et qui présente avantageusement au moins une zone de retenue ou une zone délimitée.

3. Dispositif d'injection selon l'une des revendications précédentes, dans lequel le boîtier (1) du dispositif d'injection présente au moins une arrête d'appui ou un montant (1a) sur lequel peut s'appuyer le au moins un élément de retenue (3a) dans une première position contre un coulissement en direction distale et/ou peut s'appuyer dans une deuxième position décalée en direction distale contre un coulissement en direction proximale.

4. Dispositif d'injection selon l'une des revendications précédentes, dans lequel la gaine de recouvrement (2) est placée dans le dispositif d'injection ou dans le boîtier (1) de façon à ne pas tourner par inadvertance.

5. Dispositif d'injection selon l'une des revendications précédentes avec un ressort d'injection (5) qui s'appuie contre le boîtier (1) ou contre un élément (4) associé au boîtier et qui appuie en direction distale sur la tige de piston (3).

6. Dispositif d'injection selon l'une des revendications précédentes, avec un ressort de sécurité ou un ressort de gaine de recouvrement (6) qui s'appuie contre le boîtier (1) du dispositif d'injection ou un élément (4) qui y est associé et qui génère une force de ressort ou appuie sur la sécurité ou gaine de recouvrement (2) en direction distale.

7. Dispositif d'injection selon l'une des revendications précédentes, comportant une ampoule ou une seringue pour injection (7) avec un bouchon (7a) pouvant être inséré dans celle-ci, la seringue pour injection (7) étant logée dans le boîtier (1) et/ou pouvant coulisser ou être logée dans la gaine de recouvrement (2).

8. Procédé de montage d'un dispositif d'injection à partir de deux sous-groupes d'assemblage, dans lequel un ressort d'injection (5) est tout d'abord tendu lors du montage des sous-groupes d'assemblage, le premier sous-groupe présentant un boîtier (1) et le deuxième sous-groupe présentant une tige de piston (3), un ressort d'injection (5) et un élément d'appui du ressort d'injection (4), dans lequel le ressort d'injection (5) peut être tendu ou comprimé entre la tige de piston (3) et l'élément d'appui du ressort d'injection (4).
